# EUROPEAN PATENT APPLICATION

(11) **EP 1 400 248 A1**
(43) Date of publication of application: **24.03.2004**
(21) Application number: 03020147.9
(22) Date of filing: 05.09.2003
(51) Int. Cl.: A61K 47/08, A61L 2/08

(54) **Process for sterilization of protein containing biological compositions**

(30) Priority: 19.09.2002 EP 02021305
(71) Applicant: Aventis Behring GmbH, 35002 Marburg (DE)
(72) Inventor: Lengsfeld, Thomas, 35041 Marburg (DE); Schaefer, Wolfram, 35117 Muenchhausen (DE); Nowak, Thomas, 35460 Staufenberg (DE); Grandgeorge, Michel, 69670 Vaugneray (FR)

(57) **Abstract**

The present invention relates to a method for inactivating microorganisms especially viruses in protein containing biological compositions, especially blood, placental, serum and plasma components or derivatives solutions of human or animal origin or compositions containing proteins obtained by the extraction of vegetal or animal tissues or obtained by biotechnological techniques, i.e. by culture of natural or recombinant cells of bacterial, yeast, plant or human or animal origin thereby retaining the integrity of the desired protein at a degree suitable for its purpose. By extension the method applies to the inactivation of bacterial or viral preparations, when proteic components or proteic antigens are present, which are intended for use in inactivated or non-living vaccines.

## Description

The present invention relates to a method for inactivating microorganisms especially viruses in protein containing biological compositions, especially blood, placental, serum and plasma components or derivatives solutions of human or animal origin (e.g. clotting factors, immunoglobulins, albumins, hemoglobins ...) or compositions containing proteins obtained by the extraction of vegetal or animal tissues or obtained by biotechnological techniques, i.e. by culture of natural or recombinant cells of bacterial, yeast, plant or human or animal origin (e.g. monoclonal antibodies...) thereby retaining the integrity of the desired protein at a degree suitable for its purpose. By extension the method applies to the inactivation of bacterial or viral preparations, when proteic components or proteic antigens are present, which are intended for use in inactivated or non-living vaccines.

The present invention relates also to the blood or plasma component or derivative or the protein containing composition treated by the method of the present invention as well as to the pharmaceuticals or veterinary medicines comprising the said proteins.

It is a constant concern in the manufacture of pharmaceuticals to assure that no microbial contaminants can be transmitted to the recipient or user of such products. However the proteic containing pharmaceuticals are generally sourced from biological materials which may contain viruses or their manufacturing process makes use of reagents or culture medium wich may themselves come from a biological source and therefore may be contaminated by viruses. International guidelines therefore recommend to introduce in the manufacturing processes of protein based biopharmaceuticals dedicated steps to inactivate or remove those potential viral contaminants. The European Note for Guidance CPMP/BWP/268/95 of 14 February 1996 defines what is a clearly effective method for inactivating or removing viruses as a method which is able to reduce infectivity by about 4 Log (= 4 log 10) or more.

An inherent difficulty in the task to inactivate or remove viruses in/from protein containing biological composition is due to the extreme diversity of viruses, different by their size, their structural composition as having or not an envelope (which may, in addition, differ in the relative amount of lipids), their genome which may consist of single or double stranded RNA or DNA. These and other differences explain that many known inactivating methods work well on some virus categories but not on all. For example the well known solvent-detergent method does not work on non-enveloped viruses. Also, among non-enveloped viruses some can be easily inactivated by heat or by acid pH treatment, other are resistant to heat inactivation , e.g. Parvoviruses, or to acid pH inactivation, e.g. Polioviruses.

On the other hand, therapeutic proteins are in general of complex structure and quite fragile, i.e. sensitive to degradation (modification of their primary structure) and/or denaturation (modification of their secondary, tertiary and quaternary structures), which make them difficult to withstand aggressive virus inactivating methods. These molecular modifications may result in a loss of their biological activity or antigenic properties, in a reduced stability in their pharmaceutical form upon storage, and in new immunogenicity properties which might put the recipient of such products at risk of allergic reactions upon repeated administration or application.

There is, therefore, the need of having a clearly effective virus inactivation method which would work on all categories of viruses including novel and unpredictable virus contaminants, and which would be compatible with the manufacturing of therapeutic proteins.

Light inactivation is a technology which consists in irradiating a biological composition, in a liquid or dried form, using a source of IR and/or visible light and/or UV light or X or gamma rays. In the case of low energy light, e.g. visible or UVA light, it is necessary to use the light in combination with photosensitizers like methylene blue, psoralens or riboflavin. Light inactivation occurs either by direct or indirect (through sensitizers) transfer of photon energy to the targeted biomolecule. It may also involve oxidative mechanisms due to the formation of reactive oxygen species (ROS) which may form by activation of oxygen or water during the irradiation process.

In principle light inactivation is able to preferentially target the nucleic acids rather than the proteins which make this technology attractive in the field of therapeutic proteins. This is possible because of the nucleic acid intercalating or affinity property of the photosensitizer used, or because of the preferential absorption of UVC, e.g. at a wavelength of 254 nm, by nucleic acid components in contrast to proteins, and because of the greater sensitiveness of nucleic acids to light degradation due to their larger size than protein and due to the fact that a single degradation point in their nucleotide sequence may theoretically prevent their future correct replication and therefore the multiplication of the corresponding virus.

Light inactivation technology has been tried and sometimes used since the mid 50s of last century especially in the field of blood and plasma derivatives but with mixed success until now. One major reason of this low success is that at the energy necessary to inactivate the most resistant viruses, e.g. those which have a double-stranded DNA allowing repair mechanisms, significant damage also occur to the protein (s) of interest.

Therefore, although equipment or machines now exist which would make the light inactivation technology validatable and transferable to modern transfusion institutions or pharmaceutical plants under full GMP conditions, e.g. the static mixer equipped in-flow irradiator described in GB 2 200 020, this technology is not currently used in the manufacturing of any licensed blood product or pharmaceutical, except for some particularly robust products like a crude human plasma fraction or UVC or Gamma irradiated animal serum fractions, animal peptones or some enzymes used as culture medium ingredients or as reagents in the manufacture of some cell culture derived biotechnology medicinal products or vaccines.

Some proposals have been made in order to limit the damage of proteins during light inactivation while achieving a clearly effective virus inactivation, i.e. about or more than 4 Log titer reduction:
- In US patents 6,190,608 B1 and 2001/0046450 A1 it is proposed to use a UVC tubular flow cell irradiator to inactivate non-enveloped viruses in plasma derivatives, whereby adjusting the energy applied to below 640 J/m² but at a sufficient level to retain more than 85% of the plasma derivative activity. According to the presented data, this method works well on single-stranded viruses EMC (encephalomyocarditis virus) and MVM (murine parvovirus), but failed however to inactivate sufficiently the enveloped double-stranded virus BHV (2,5 to 3.0 Log reduction only in various plasma derivatives).
- In US patent 5,981,163 it is proposed to protect proteins against oxidative damage during irradiation using a combination of ROS quenchers of type I (quenchers of free radical ROS species) and ROS quenchers type II (quenchers of the ROS molecule singlet oxygen), or to use molecules which quench both ROS types. As examples of quenchers type I or type II the following compounds are claimed: mannitol, glycerol, glutathione, SOD (Superoxide Dismutase) (all type I quenchers), histidine, tryptophane (both type II quenchers) and ascorbate (not indicated if a type I or type II). As examples of quenchers which quench both type I and type II only flavonoid compounds are claimed, in particular rutin. Rutin is given as the most effective protectant of all, more effective than the combination of type I and type II quenchers. The inventors confirmed their preference of rutin in several publications where they report on the use of UVC inactivation on plasma and various plasma derivatives (Factor VIII, albumin, immunoglobulins and fibrinogen) - See for instance Photochemistry and Photobiology 1997, 65(3): 432-435 - However, the authors of the 1997 paper report a reduction of the inactivation of EMC virus in albumin in presence of 1.6 mmol/lrutin and no significant protection at this concentration of rutin against albumin degradation/denaturation as evidenced by the formation of albumin dimers and larger aggregates. The removal of these aggregates was performed in a subsequent purification step by filtration through a Sephacryl S200 HR column. On the other hand, rutin is a complex molecule of large molecular weight (610 Da), is poorly soluble in aqueous solutions and therefore difficult to remove from the product after the inactivation step. In addition, rutin as been identified as a mutagenic substance, which might be a serious concern for use in the production of medicinal products - See Mutation Research 1986, 170: 103-113.

The present invention aims to obtain a new sterilization. method for inactivating microorganisms especially viruses of all kind, enveloped and non-enveloped viruses, in a clearly effective way, i.e. with about 4 Log or more virus reduction, in protein containing composition while minimizing the damage to the protein (s) of interest, and without the disadvantages inherent to the existing methods.
An other aim is to improve the overall safety of protein containing pharmaceuticals for human or animal use, by including in their manufacturing process the new sterilization method.

It has been found surprisingly that a light inactivation process could be applied on protein containing compositions with an energy sufficient to obtain a virus reduction factor of 4 Log with enveloped viruses as well as with non-enveloped viruses, including viruses more resistant to light inactivation, i.e. the double-stranded DNA viruses, under the condition that proteins are protected during the irradiation process by a substance of the general formula (I) 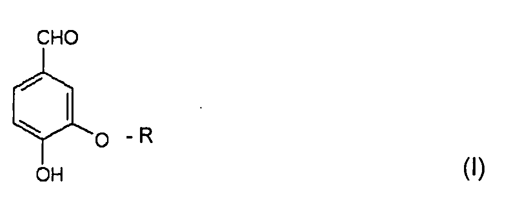 wherein R=H,CH3 or C2H5.

A substance of very good effect is vanillin (R=CH3). Vanillin is a flavouring agent largely used in confectionery, beverages, foods, galenicals and perfumery. It is a simple molecule of 152 Da, readily soluble in aqueous solutions and classified as a GRAS (Generally Considered As Safe).

Thus the present invention relates to a method for sterilizing a protein containing biological composition, said method comprising the step of subjecting said composition to a virucidally effective amount of artificial irradiation in the presence of a substance of the general formula (I) 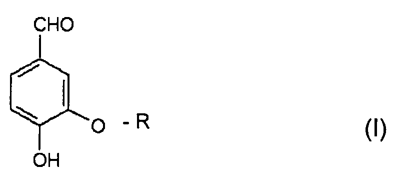 wherein R=H,CH3 or C2H5

It also relates to the method wherein at least one enveloped double-stranded DNA-virus and at least one non-enveloped single-stranded DNA-virus is inactivated by at least 4 Log.

It also relates to the method wherein the irradiation is UV, IR, gamma-irradiation, x-ray or visible light.

It also relates to the method, wherein in formula (I) R=CH3 (vanillin).

It also relates to the method, wherein the irradiation is UVA, UVB or UVC.

It also relates to the method, wherein the irradiation is UVC at a wavelength of 240 to 290 nm.

It also relates to the method, wherein the irradiation is UVC at a wavelength of 254 nm.

It also relates to the method, wherein said protein containing biological composition contains purified plasma proteins.

It also relates to the method, wherein said plasma protein is a coagulation factor.

It also relates to the method, wherein said coagulation factor is selected from the group consisting of factors V, VII, VIII, IX, X, XI and XIII and fibrinogen.

It also relates to the method, wherein the coagulation factor is factor VIII.

It also relates to the method, wherein said plasma protein retains at least 85% of its biological activity after treatment with irradiation.

It also relates to the method, wherein said plasma protein retains at least 95% of its biological activity after treatment with irradiation.

It also relates to the method, wherein not more than 5 % of aggregates are formed during irradiation.

It also relates to the method, wherein either before, after or at the same time as said protein containing biological composition is subjected to said irradiation and said compound of general formula (I), the composition is subjected to at least one different virucidal method.

It also relates to the method, wherein the different virucidal method is selected from the group consisting of heat treatment, pH manipulation, solvent or detergent or and detergent treatment, and gamma-irradiation treatment.

It also relates to the method, wherein the substance of the general formula (I) is employed in a concentration of 0.1 to 25 mmol/l.

It also relates to the method, wherein the substance of the general formula (I) is employed in a concentration of 0.5 to 5 mmol/l.

It also relates to the method, wherein factor VIII is associated with von Willebrand factor.

It also relates to a method of using a substance of general formula (I) in an virus-inactivation process.

It further relates to a pharmaceutical composition for the use with humans or animals, containing at least one ingredient, sterilized by the method according to claim 1.

And it relates to a pharmaceutical product for the use with humans or animals, for the production of which the inventive method for sterilizing a protein containing biological composition has been used.

Vanillin was compared to rutin in various experiments using a UV in-flow irradiator similar to the one described in GB 2 200 020 and a fibrinogen containing composition and a factor VIII containing composition as models for fragile proteins. Degradation/denaturation of these proteins was evaluated by measuring their biological activity as well as their aggregates content before and after irradiation. Virus inactivation was evaluated in spiking experiments with various model viruses especially a parvovirus - CPV = canine parvovirus- (a non-enveloped virus with a single-strain DNA) and an herpes virus -PRV = pseudorabies virus- (enveloped, with a double-stranded DNA). In these experiments, vanillin compared better to rutin, especially allowing a higher Log inactivation number of the most resistant virus PRV. In addition vanillin has not the drawbacks of rutin in terms of structure complexity, low water solubility, and concerns regarding mutagenicity. The optimal range of vanillin concentrations was established in the fibrinogen containing composition and found to be between 1 and 2 mM. This range needs to be verified similarly for each protein containing composition on the model of what was done with the fibrinogen containing solution. Some Type I or Type II quenchers suggested in US patent 5,981,163 as possible stabilizers where also tested alone or in combination, without any success.

Vanillin shows here exceptional properties in protecting proteins during an irradiation process which cannot be simply explained by a Type I/TypeII ROS quenching theory because known Type I and Type II either alone or in combination were not found effective.

The following examples will describe, without limiting the scope of the invention, in more details conditions and results of all these experiments.

### Example 1

Fibrinogen (5 g/L fibrinogen, buffered in 50 mmol/lNaCl, 20 mmol/lNaCitrate , pH 7.3 solution) non stabilised or stabilised with 0.5 mM rutin was treated with a UV in-flow irradiator equipped with a static mixer as described in GB 2 200 020.
The non stabilised and stabilised solutions were spiked with PRV for a final concentration of 7 Log CCID₅₀/ml or with CPV for a final concentration of 7 Log CCl D₅₀/ml.
The UVC illuminated tube of the irradiator had a 6 mm diameter and a length of 35 cm. The illuminated tube was surrounded by four UVC lamps (15 Watt each, 40 cm length, Philips TUV 16 W, Netherlands) providing a maximal emission of 254 nm. The materials were pumped with a flow rate of 250 ml/min and re-circulated eight times through the irradiator.

After irradiation it was found, that the PRV titer was reduced by 4.5 Log , and the CPV titer was reduced to non detectable (i.e. more than 7 Log reduction) in the non stabilised solution. In the rutin stabilised solution the PRV titer was reduced by 3 Log and CPV titer was reduced to non detectable (i.e. more than 7 Log reduction).

Protein aggregate formation was studied by SEC (Size Exclusion Chromatography) using
a TSK G 4000SWXL column, TosoHaas, Japan.
22 % new aggregates were found in the non stabilised solution after treatment. In the rutin stabilised solution only 1 % new aggregates were found.

### Example 2

Fibrinogen (5 g/L fibrinogen, buffered in 50 mmol/I NaCl, 20 mmol/I NaCitrate, pH 7.3 solution) non stabilised or stabilised with mannitol as mentioned in US 5,981,163 at two concentrations (2 mmol/l; 100 mM) in two separate experiments was treated with the irradiator and the same irradiation conditions as described in example 1.
The materials were pumped six times through the irradiator.

Protein aggregate formation was measured as described in example 1.
18 and 20 % respectively new aggregates were found after treatment in the non stabilised solutions.
7 % new aggregates were found after treatment in the 2 mmol/l mannitol stabilised solution. 9 % new aggregates were found after treatment in the 100 mmol/I mannitol stabilised solution.

### Example 3

Fibrinogen (5 g/L fibrinogen, buffered in 50 mmol/l NaCl, 20 mmol/I NaCitrate, pH 7.2 solution) non stabilised or stabilised with histidine as mentioned in US 5,981,163 at two concentrations (2 mmol/l; 5 mM) was treated with the irradiator and the same irradiation conditions as described in example 1.
The materials were pumped six times through the irradiator.

Protein aggregate formation was measured as described in example 1.
20 % new aggregates were found after treatment in the non stabilised solutions. 10 % new aggregates were found after treatment in the 2 mmol/I histidine stabilised solution. 12 % new aggregates were found after treatment in the 5 mmol/I histidine stabilised solution.

### Example 4

Fibrinogen (5 g/L fibrinogen, buffered in 50 mmol/I NaCl, 20 mmol/l NaCitrate, pH 7.3 solution) non stabilised or stabilised with a mixture of histidine and mannitol as suggested in US 5,981,163 at the following concentrations: 2 mmol/l histidine and 100 mmol/I mannitol, was treated with the irradiator and the same irradiation conditions as described in example 1.
The materials were pumped six times through the irradiator.

Protein aggregate formation was measured as described in example 1.
20 % new aggregates were found after treatment in the non stabilised solutions. 9 % new aggregates were found after treatment in the histidine + mannitol stabilised solution.

### Example 5

Fibrinogen (5 g/L fibrinogen, buffered in 50 mmol/I NaCl, 20 mmol/I NaCitrate, pH 7.3 solution) non stabilised or stabilised with different concentrations of vanillin (0.01; 0.05; 0.1; 0.5; 1 : 2; 3 and 4 mM) was treated with the irradiator and the same irradiation conditions as described in example 1.
The materials were pumped eight times through the irradiator.

Protein aggregate formation was measured as described in example 1.
20 % new aggregates were found after treatment in the non stabilised solutions. 13 % new aggregates were found after treatment in the 0.01 mmol/l vanillin stabilised solution.
9 % new aggregates were found after treatment in the 0.05 mmol/l vanillin stabilised solution.
7 % new aggregates were found after treatment in the 0.1 mmol/l vanillin stabilised solution.
3 % new aggregates were found after treatment in the 0.5 mmol/l vanillin stabilised solution.
2 % new aggregates were found after treatment in the 1 mmol/l vanillin stabilised solution.
1 % new aggregates were found after treatment in the 2 mmol/I vanillin stabilised solution.
1 % new aggregates were found after treatment in the 3 mmol/l vanillin stabilised solution.
1 % new aggregates were found after treatment in the 4 mmol/l vanillin stabilised solution.

### Example 6

Fibrinogen (5 g/L fibrinogen, buffered in 50 mmol/l NaCl, 20 mmol/I NaCitrate , pH 7.3 solution) non stabilised or stabilised with 1 mmol/l or 2 mmol/I vanillin was treated with a UV in-flow irradiator and under conditions as described in example 1.

The non stabilised and stabilised solutions were spiked with PRV for a final concentration of 7 Log CCID₅₀/ml or with CPV for a final concentration of 7 Log CClD₅₀/ml.
The materials were pumped with a flow rate of 250 ml/min and recirculated eight times through the irradiator.

After irradiation it was found, that the PRV titer was reduced by 4.5 Log , and the CPV titer was reduced to non detectable (i.e. more than 7 Log reduction) in the non stabilised solution. In the vanillin stabilised solutions the PRV titer was reduced by 5.5 Log (1mmol/l and 2 mmol/l vanillin) and CPV titer was reduced to non detectable (1mmol/l and 2 mmol/I vanillin) which corresponds to a reduction factor of more than 7 Log.

### Example 7

Fibrinogen (5 g/L fibrinogen, buffered in 50 mmol/I NaCl, 20 mmol/l NaCitrate, pH 7.3 solution) non stabilised or stabilised with vanillin or rutin at three concentrations (0.5 mM, 1 mmol/I and 2 mM).
The non stabilised and rutin or vanillin stabilised solutions were spiked with PRV for a final concentration of 7 Log CCID₅₀/ml or with CPV for a final concentration of 7 Log CCID₅₀/ml.
The solutions were treated with the irradiator and the same irradiation conditions as described in example 1.

The materials were pumped eight times through the irradiator.

Protein aggregate formation was measured as described in example 1. Results see Tab.1:

**Tab.1:**

| Results Example 7 | | | | | | | |
|---|---|---|---|---|---|---|---|
| Stabiliser and concentration | 0 | 0.5 mM vanillin | 0.5 mmol/I rutin | 1 mmol/l vanillin | 1 mmol/I rutin | 2 mM vanillin | 2 mmol/l rutin |
| Log reduction PRV | 4.5 | 5 | 3 | 5.5 | 3 | 5.5 | 2.5 |
| Log reduction CPV | >7 | >7 | >7 | >7 | >7 | >7 | 7 |
| % aggregate increase | 20 | 3 | 1 | 2 | 1 | 1 | 1 |

### Example 8

Factor VIII (in 5 g/L Protein solution, buffered a Citrate/NaCl/Glycine, pH 7.3 solution) non stabilised or stabilised with 2 mmol/I vanillin. The non stabilised and the 2 mmol/I vanillin stabilised solutions were spiked with PRV for a final concentration of 7 Log CCID₅₀/ml.
The solutions were treated with the irradiator and under conditions as described in example 1.

The materials were pumped six times through the device.

FVIII activity was measured by a FVIII chromogenic assay. A 50 % decrease in FVIII activity was found in the non stabilised solution after treatment.
A 10 % decrease in FVIII activity was detected after the treatment in presence of 2 mmol/l vanillin.

After irradiation it was found, that the PRV titer was reduced by 4 Log in the non stabilised solution. In the 2 mmol/l vanillin stabilised solution the PRV titer was reduced by 5 Log

## Claims

1. A method for sterilizing a protein containing biological composition, said method comprising the step of subjecting said composition to a virucidally effective amount of artificial irradiation in the presence of a substance of the general formula (I) 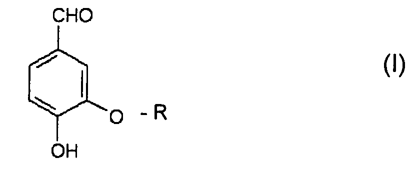 wherein R=H,CH3 or C2H5

2. The method of claim 1, wherein at least one enveloped double-stranded DNA-virus and at least one non-enveloped single-stranded DNA-virus is inactivated by at least 4 Log.

3. The method of claim 1 or 2, wherein the irradiation is UV, IR, gamma-irradiation, x-ray or visible light.

4. The method of any of claims 1 to 3, wherein in formula (I) R=CH3 (vanillin).

5. The method of any of claims 1 to 4, wherein the irradiation is UVA, UVB or UVC.

6. The method of any of claims 1 to 5, wherein the irradiation is UVC at a wavelength of 240 to 290 nm.

7. The method of any of claims 1 to 6, wherein the irradiation is UVC at a wavelength of 254 nm.

8. The method of any of claims 1 to 7, wherein said protein containing biological composition contains purified plasma proteins.

9. The method of claim 8, wherein said plasma protein is a coagulation factor.

10. The method of claim 9, wherein said coagulation factor is selected from the group consisting of factors V, VII, VIII, IX, X, XI and XIII and fibrinogen.

11. The method of claim 10, wherein the coagulation factor is factor VIII.

12. The method of claim 8, wherein said plasma protein retains at least 85% of its biological activity after treatment with irradiation.

13. The method of claim 8, wherein said plasma protein retains at least 95% of its biological activity after treatment with irradiation.

14. The method of claim 8, wherein not more than 5 % of aggregates are formed during irradiation.

15. The method of claim 1, wherein either before, after or at the same time as said protein containing biological composition is subjected to said irradiation and said compound of general formula (I), the composition is subjected to at least one different virucidal method.

16. The method of claim 15, wherein the different virucidal method is selected from the group consisting of heat treatment, pH manipulation, solvent or detergent or and detergent treatment, and gamma-irradiation treatment.

17. The method of claim 1, wherein the substance of the general formula (I) is employed in a concentration of 0.1 to 25 mmol/l.

18. The method of claim 17, wherein the substance of the general formula (I) is employed in a concentration of 0.5 to 5 mmol/l.

19. The method of claim 11, wherein factor VIII is associated with von Willebrand factor.

20. The method of using a substance of general formula (I) in an virus-inactivation process.

21. A pharmaceutical composition for the use with humans or animals, containing at least one ingredient, sterilized by the method according to claim 1.

22. A pharmaceutical product for the use with humans or animals, for the production of which the method of claim 1 has been used.
